# EUROPEAN PATENT APPLICATION

(11) **EP 4 772 196 A2**
(43) Date of publication of application: **08.07.2026**
(21) Application number: 26179026.5
(22) Date of filing: 25.09.2023
(51) Int. Cl.: A61K 47/02

(54) **NGF FOR THE TREATMENT OF SPASTICITY**

(30) Priority: 23.09.2022 EP 22197562
(62) Divisional of application: 23777211.6
(71) Applicant: Dompé farmaceutici SpA, 20122 Milano (IT)
(72) Inventor: ALLEGRETTI, Marcello, 67100 L'Aquila (IT); ARAMINI, Andrea, 67100 L'Aquila (IT); BRANDOLINI, Laura, 67100 L'Aquila (IT); CATTANI, Franca, 67100 L'Aquila (IT); MANTELLI, Flavio, 67100 L'Aquila (IT)
(74) Representative: Dompé farmaceutici Spa

(57) **Abstract**

The present invention relates to nerve growth factor (NGF) or a mutein thereof for use in the prevention or treatment of spasticity in a subject, wherein preferably said NGF or mutein is administered intranasally to said subject.

## Description

### FIELD OF THE INVENTION

The present invention relates to the prevention or treatment of spasticity in a subject.

### STATE OF THE ART

Spasticity is a motor disorder characterised by a velocity dependent increase in muscle tone, with exaggerated muscle tendon jerks that is caused by hyperexcitability of the muscle stretch reflex (J.W. Lance, "Symposium synopsis," in Spasticity: Disordered Motor Control, R. G. Feldman, R. R. Young, and W. P. Koella, Eds., pp. 485-494, 1980). It can arise as a consequence of traumatic insults, chronic neurodegenerative conditions or genetic defects that cause the disruption or malfunctioning of the areas of the brain and nerve tracks responsible for controlling the initiation and modulation of movements. These include, for example, cerebral palsy, stroke, traumatic brain injury, amyotrophic lateral sclerosis, primary lateral sclerosis, multiple sclerosis (MS) and hereditary spastic paraplegia (HSP) (Mathewson et al, Phys Med Rehabil Clin N Am 2015, 26(1): 57-67; Khundadze et al, Autophagy 2021, 17(11): 3690-3706; Chang et al, Crit Rev Phys Rehabil Med 2013, 25(1-2): 11-22).

Spasticity is highly invalidating condition for patients as it affects their ability to conduct basic daily activities such as hygiene, dressing, ambulation and sleep. Without therapy, in the long-term spasticity can lead to pain, permanent joint deformity, urinary tract infections, chronic constipation, peripheral neuropathy and pressure ulcers.

Therefore, treatment of this condition is imperative to improve the quality of life and avoid serious medical complication.

Currently available treatment options are limited and are aimed at only palliating the symptoms to allow individuals to live with the least amount possible of discomfort and restriction, without a significant number of side effects.

Available treatments include systemic medications and interventional procedures.

Systemic medications comprise centrally acting agents, such as baclofen, anticonvulsants such as benzodiazepines and gabapentin and peripherally acting agents such as dantrolene. Interventional procedures include focal injections of botulinum toxin, phenol or alcohol, and the insertion of an intrathecal baclofen pump.

Unfortunately, all treatments available are characterized by adverse effect and risks which can outweigh the potential benefits they may provide. Systemic drugs may be better for patients with generalized spasticity but can cause a number of unwanted effects such as systemic muscle relaxation, sedation, fatigue and in some cases, produce tolerance and dependence.

Interventional therapies are generally associated with fewer systemic side effects if patients are compliant and the procedures are performed correctly. However, they need to be performed by an appropriately trained professional as they may result in serious complications in case of procedural errors, for example resulting from dissemination of the drug to other areas of the body.

It is therefore strongly felt the need of developing new effective, long lasting and safe therapeutic approaches for the prevention or treatment of spasticity. Furthermore, it is felt the need of identifying new treatments that address not only the symptoms of this conditions but also the underlying pathological mechanism.

### SUMMARY OF THE INVENTION

As it will be described in the experimental section, the present inventors have found that when NGF is administered intranasally, it is delivered at particularly high concentrations to the areas of the brain that are compromised in patients with spasticity.

The present inventors have also surprisingly found that the intranasal administration of NGF is effective in restoring the activity of these areas, thereby decreasing spasticity and consequently improving motor ability of the patient.

Furthermore, the efficacy of intranasally administered NGF on spasticity is independent from the nature of the underlying cause inducing spasticity. In fact, the inventors have found that NGF restores motor activity in animal models of both mechanically induced brain damage (TBI model) and in physiopathological damage of relevant neuronal cells (chemogenetic model).

On the basis of the data obtained, contrary to the available therapeutic options available to date, NGF is therefore able to decrease the symptomatology of patients affected by spasticity, and to at least partially reverse the motor impairment associated thereof. Accordingly, object of the invention is NGF or a mutein thereof for use in the prevention or treatment of spasticity in a subject.

### BRIEF DESCRIPTION OF THE FIGURES

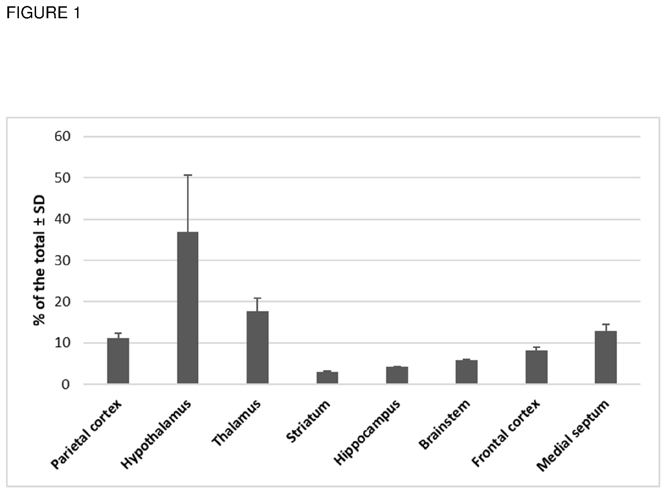

Figure 1 shows NGF biodistribution in rat brain areas after intranasal administration, measured by ELISA over 24 hours, as described in Example 1. Results are expressed as percent of the total amount absorbed over 24 hours in the rat brain after administration.

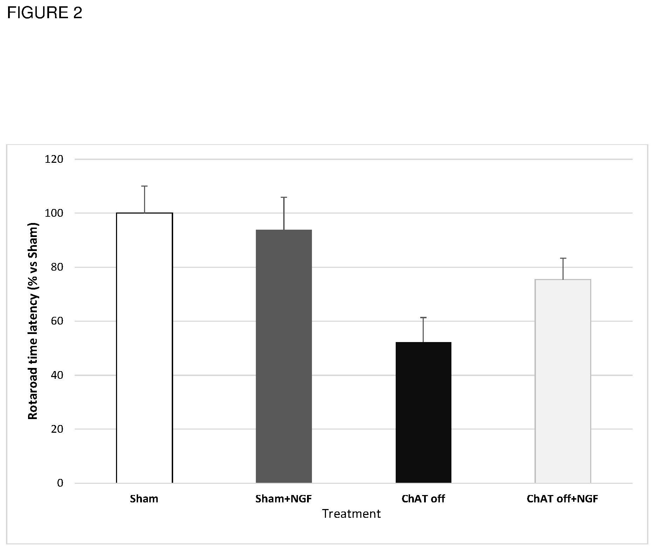

Figure 2 shows the "rotarod time latency" (% vs sham animals) in rats in which cholinergic neurons were chemogenetically blocked (ChAT off) or in sham rats (Sham), treated with vehicle (ChAT off and Sham, respectively) or with rhNGF by intranasal administration (ChAT off+NGF and Sham+NGF, respectively), as described in Example 2.

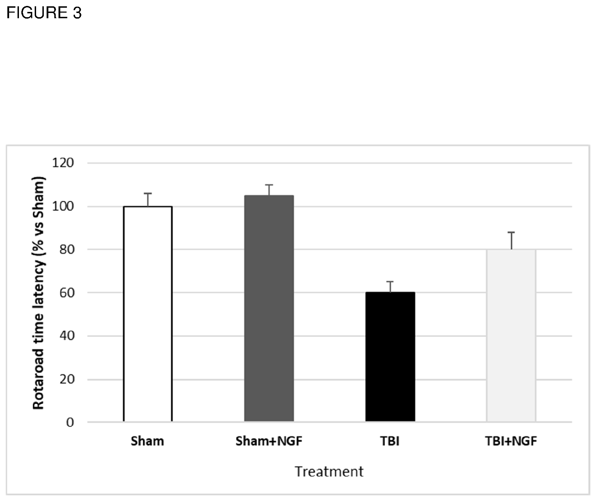

Figure 3 shows the "rotarod time latency" (% vs sham animals) in mice with Traumatic Brain Injury or sham, treated with vehicle (TBI or Sham, respectively) or with rhNGF (TBI+NGF and Sham+NGF, respectively) by intranasal administration, as described in Example 3.

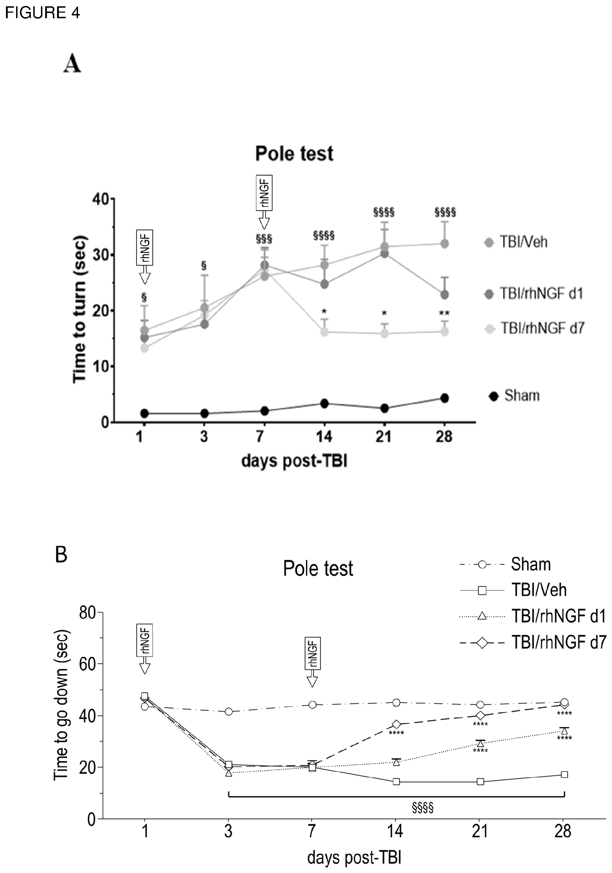

Figure 4 shows the times, expressed in seconds, required to turn (panel A) and descend (panel B) from the pole for sham mice (Sham) or mice with traumatic brain injury, treated by intranasal administration with vehicle (TBI/Veh) or rhNGF, starting at day 1 (TBI/rhNGF d1) or day 7 (TBI/rhNGF d7), after TBI, as described in Example 4. §P < 0.05, §§P < 0.01, §§§P < 0.001 and §§§§P < 0.0001 vs Sham and, *P < 0.05, **P <0.01, ****P < 0.0001 vs TBl/vehicle. Data are expressed as means ± SEM (n = 20/group).

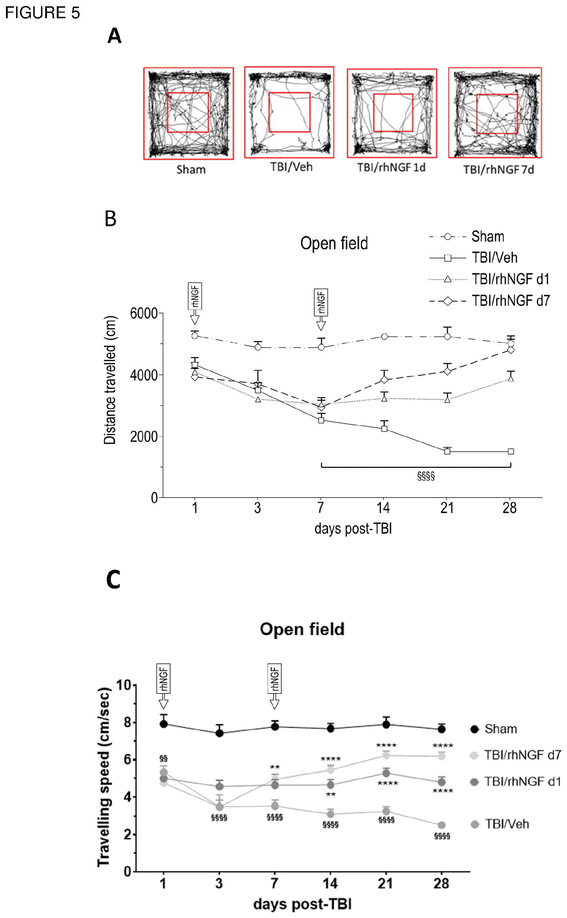

Figure 5 shows results of the open field test on sham mice (Sham) or mice with traumatic brain injury, treated by intranasal administration with vehicle (TBI/Veh) or rhNGF, starting at day 1 (TBI/rhNGF d1) or day 7 (TBI/rhNGF d7), after TBI, as described in Example 4. In details, panel A show representative trajectory diagrams; panel B shows average distance travelled, expressed in cm; panel C shows travelling speed, expressed in cm/sec. §§P < 0.01, §§§P < 0.001 and §§§§P < 0.0001 vs Sham, **P <0.01, ***P <0.001 and ****P < 0.0001 vs TBl/vehicle. Data are expressed as means ± SEM (n = 20/group).

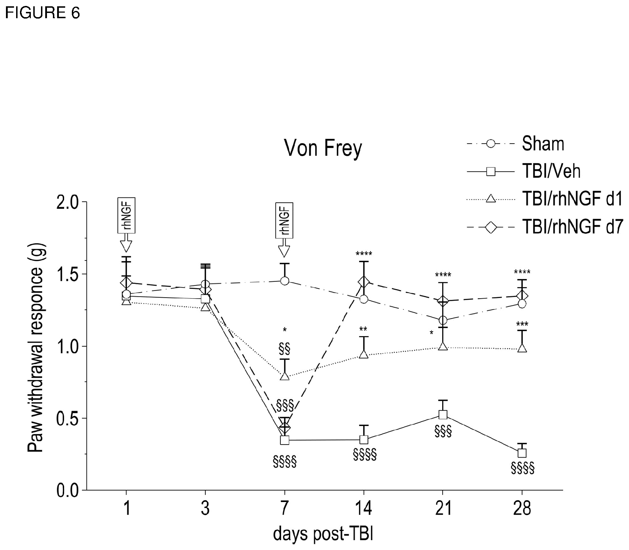

Figure 6 shows mechanical allodynia, expressed as paw withdrawal response (g), measured in sham mice (Sham) or mice with traumatic brain injury, treated by intranasal administration with vehicle (TBI/Veh) or rhNGF, starting at day 1 (TBI/rhNGF d1) or day 7 (TBI/rhNGF d7), after TBI, as described in Example 4. §§P < 0.01, §§§P < 0.001 and §§§§P < 0.0001 vs Sham, *P <0.05, **P <0.01 and ****P < 0.0001 vs TBl/vehicle. Data are expressed as means ± SEM (n = 20/group).

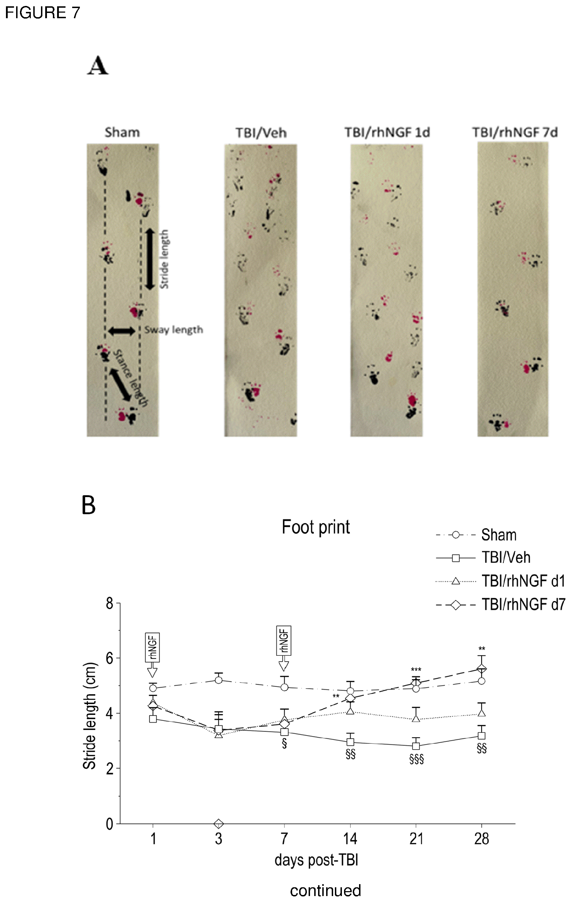

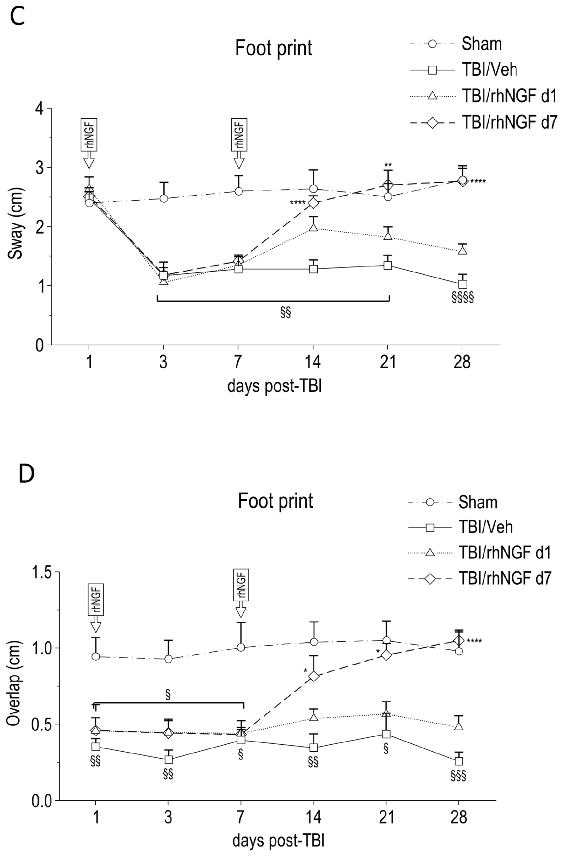

Figure 7 shows the results of the footprint test on sham mice (Sham) or mice with traumatic brain injury, treated by intranasal administration with vehicle (TBI/Veh) or rhNGF, starting at day 1 (TBI/rhNGF d1) or day 7 (TBI/rhNGF d7), after TBI, as described in Example 4. In details, panel A shows footprints recordings and parameters measured in footprint analysis with dotted lines representing the direction of progression (DoP) of walking: vertical black arrow indicates stride length analysis, horizontal black arrow indicates the sway length and the oblique black arrow indicates the overlap; panel B stride length, measured in cm; panel C shows sway length, measured in cm; panel D shows overlap, measured in cm. §P < 0.05, §§P < 0.01, §§§P < 0.001 and §§§§P < 0.0001 vs Sham, *P < 0.05, **P <0.01 and ****P < 0.0001 vs TBl/vehicle. Data are expressed as means ± SEM (n = 10/group).

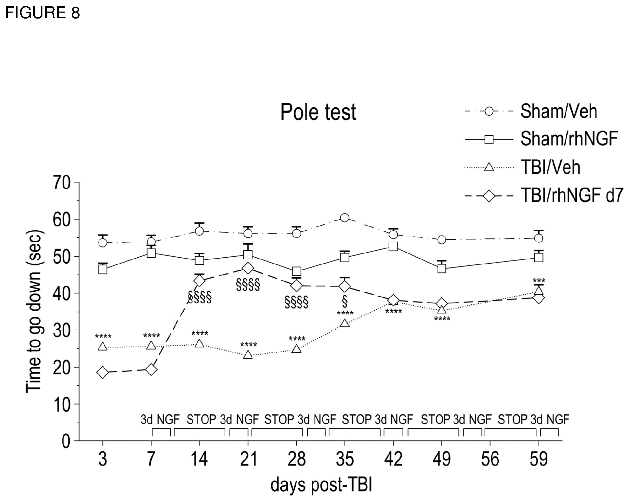

Figure 8 shows the times, expressed in seconds, required to descend from the pole for sham mice (Sham) or mice with traumatic brain injury, treated by intermittent intranasal administration with vehicle (TBI/Veh) or rhNGF from day 7 (TBI/rhNGF d7), after TBI, as described in Example 5.

### DETAILED DESCRIPTION OF THE INVENTION

A first object of the invention is nerve growth factor (NGF) or a mutein thereof for use in the prevention or treatment of spasticity in a subject.

Preferably, said NGF or mutein is administered intranasally to said subject.

Preferably, said spasticity is spasticity caused by a condition selected from cerebral palsy, stroke, traumatic brain injury, amyotrophic lateral sclerosis, primary lateral sclerosis, multiple sclerosis (MS) and hereditary spastic paraplegia (HSP).

The terms "treatment" and "prevention" as used herein refer to the eradication/amelioration or prevention/delay in onset, respectively, of a disorder or of one or more of the symptoms associated thereof.

Preferably, said subject is a human subject.

According to one embodiment, said subject has been diagnosed with spasticity caused by a condition selected from cerebral palsy, stroke, traumatic brain injury, amyotrophic lateral sclerosis, primary lateral sclerosis, multiple sclerosis (MS) and hereditary spastic paraplegia (HSP) and said NGF or mutein thereof is for use in the treatment of said spasticity in said subject, preferably by intranasal administration to said subject. According to an alternative embodiment, said subject has been identified as being at risk of developing spasticity caused by a condition selected from cerebral palsy, stroke, traumatic brain injury, amyotrophic lateral sclerosis, primary lateral sclerosis, multiple sclerosis (MS) and hereditary spastic paraplegia (HSP) and said NGF or mutein thereof is for use in the prevention of said spasticity in said subject prior to the development of said spasticity, preferably by intranasal administration to said subject.

Preferably said NGF is human NGF.

Preferably, said human NGF has the aminoacid sequence of SEQ ID NO:1 below

SEQ. ID NO:1:
SSSHPIFHRGEFSVCDSVSVWVGDKTTATDIKGKEVMVLGEVNINNSVFKQYFFETKC
RDPNPVDSGCRGIDSKHWNSYCTTTHTFVKALTMDGKQAAWRFIRIDTACVCVLSRK
AVR

Alternatively, said human NGF has the aminoacid sequence of SEQ ID NO:2 below:

SEQ ID NO:2:
SSSHPIFHRGEFSVCDSVSVWVGDKTTATDIKGKEVMVLGEVNINNSVFKQYFFETKC
RDPNPVDSGCRGIDSKHWNSYCTTTHTFVKALTMDGKQAAWRFIRIDTACVCVLSRK
AVRRA

Alternatively, said human NGF is a mixture of NGFs having sequences of SEQ ID NO:1 and SEQ ID NO:2.

The human NGF of SEQ ID NO:2 has an amino acid sequence that only differ from the NGF of SEQ ID NO: 1 for the presence of two additional amino acids at the C-terminus. Both forms of NGF are found in human cells and therefore are considered as wild type human NGF.

Therefore, when referring to "human NGF" or "wild type human NGF" in the present application, it is meant a human NGF of SEQ ID NO:1 or of SEQ ID NO:2.

Among the two forms of wild type NGF, the human NGF of SEQ ID NO:1 is particularly preferred. In fact, it has been found by the inventors that this specific form of NGF has particularly advantageous biological activity compared to the NGF of SEQ ID NO:2, showing a higher neuroprotective activity on neuronal cells.

Preferably, said NGF is produced by recombinant DNA technology, preferably it is a human recombinant NGF (rhNGF). Methods of producing rhNGF are known to the person skilled in the art, for example those described in WO0022119A1 and WO2013092776A1. Preferably, said NGF has a purity higher than 70%, more preferably higher than 80%, higher than 90%, higher than 95%, higher than 98% or higher than 99%. The purity of NGF may be determined by conventional means known to those skilled in the art, for example by HPLC analysis.

The term "mutein of NGF" refers to a biologically active mutein of NGF, meaning a NGF protein having an amino acid sequence with one or more amino acid mutations, preferably substitutions, such that the therapeutic activity of wild type NGF is maintained. Preferably, said mutein is a mutein of wild type human NGF, as defined above.

Particularly preferred for use according to the invention, is a mutein of NGF, wherein the mutein is characterized by more than 80 %, more preferably more than 90 %, even more preferably more than 95%, and most preferably more than 98% sequence identity with wild type human NGF.

Preferably, the mutein is a mutein of wild type human NGF characterized by at least one mutation, preferably the amino acid substitution of proline at position 61 by another amino acid, in the sequence of wild type human NGF. In a particularly preferred embodiment, proline at position 61 is substituted by serine.

Preferably, the mutein is a mutein of human NGF, characterized by at least one mutation of the amino acid sequence associated with reduced nociceptive activity. More preferably said mutein is characterized by at least one mutation, preferably amino acid substitution, at any of positions 95-101 of wild type human NGF. Even more preferably, said mutein is characterized by the substitution of the arginine in position 100 of wild type human NGF by another amino acid. More preferably, arginine at position 100 of wild type human NGF is substituted by glutamic acid.

Preferably, the mutein is a mutein of wild type human NGF characterized by at least the amino acid substitution of proline at position 61 by another amino acid, preferably serine, and the substitution of the arginine in position 100 of wild type human NGF by another amino acid, preferably glutamic acid.

Particularly preferred muteins according to the invention have the amino acid sequences of SEQ ID NO:3-6 below
SEQ ID NO:3: 
SSSHPIFHRGEFSVCDSVSVWVGDKTTATDIKGKEVMVLGEVNINNSVFKQYFFETKC
RDPNPVDSGCRGIDSKHWNSYCTTTHTFVKALTMDGKQAAWEFIRIDTACVCVLSRKA
VR
SEQ ID NO:4: 
SSSHPIFHRGEFSVCDSVSVWVGDKTTATDIKGKEVMVLGEVNINNSVFKQYFFETKC
RDPNPVDSGCRGIDSKHWNSYCTTTHTFVKALTMDGKQAAWEFIRIDTACVCVLSRKA
VRRA
SEQ ID NO:5: 
SSSHPIFHRGEFSVCDSVSVWVGDKTTATDIKGKEVMVLGEVNINNSVFKQYFFETKC
RDSNPVDSGCRGIDSKHWNSYCTTTHTFVKALTMDGKQAAWEFIRIDTACVCVLSRKA
VR
SEQ ID NO:6:

SSSHPIFHRGEFSVCDSVSVWVGDKTTATDIKGKEVMVLGEVNINNSVFKQYFFETKC
RDSNPVDSGCRGIDSKHWNSYCTTTHTFVKALTMDGKQAAWEFIRIDTACVCVLSRKA
VRRA

The above described muteins are particularly advantageous for use according to the invention since they have been described to maintain the same bioactivity than wild type human NGF but to be able to induce a lower nociceptive sensitivity compared to the corresponding wild type human NGF. Preferably, said mutein of human NGF is produced by recombinant DNA technology. Methods of producing muteins of rhNGF according to the invention by recombinant DNA technology are known to the person skilled in the art, for example those described in WO2019/207106.

Preferably, the NGF or mutein for use according to the invention is administered to the subject daily or every two/three days throughout the period of treatment.

Preferably, said period of treatment of between 7 and 300 days, preferably between 60 and 240 days, more preferably between 100 and 200 days.

More preferably, the NGF or mutein for use according to the invention is administered to the subject from one to three times a day for a period of treatment of between 7 and 300 days, preferably between 60 and 240 days, more preferably between 100 and 200 days. A preferred administration schedule, is a continuous administration schedule, wherein said NGF or mutein is administered according to the same schedule for said period of treatment.

Alternatively, several cycles of treatment may be performed. A preferred administration schedule according to this is an intermittent administration schedule, with two or more cycles of periods of treatment alternated by wash-out periods.

The present inventors have observed that, surprisingly, in case of spasticity induced by a traumatic event, such as stroke or traumatic brain injury, treatment with NGF is more effective if it is started once spasticity symptoms have fully developed. Accordingly, when said nerve growth factor (NGF) or mutein thereof is for use in the treatment of spasticity caused by a condition selected from stroke or traumatic brain injury in a subject, said NGF or mutein is preferably administered to the subject starting between 6 and 10 months, more preferably between 8 and 10 months, more preferably between 9 and 10 months, even more preferably at 9 months after from the occurrence of said stroke or traumatic brain injury. Preferably, in all above preferred embodiments said NGF or mutein is administered intranasally to said subject.

Preferably, when NGF or mutein thereof is administered intranasally, the amount of NGF or mutein thereof per each administration is between 5 µg and 1 mg, more preferably between 10 µg and 400 µg, even more preferably between 15 µg and 200 µg.

The effective amount of said NGF or mutein used in each administration, the duration of the treatment and the number of administrations for day are selected by the skilled person on the basis of the characteristics of the subject to be treated, the severity of the spasticity and on the basis of assessment tests carried out during the treatment.

A further object of the present invention relates to a pharmaceutical composition for intranasal administration comprising the NGF or mutein as described above and at least one pharmaceutically acceptable excipient suitable for intranasal use.

Preferably, the pharmaceutical composition for intranasal administration of the invention is a liquid intranasal composition.

Preferably, the pharmaceutical composition according to the present invention comprises an effective amount of the NGF or mutein as described above and at least one pharmaceutically acceptable excipient suitable for intranasal use, preferably selected from solvents, thickening agents, mucoadhesive agents, buffers, antioxidants, preservatives, and penetration enhancers.

Preferably, the concentration of said NGF or mutein in the liquid intranasal composition according to the invention is between 5 µg/ml and 1 mg/ml, more preferably between 10 µg/ml and 400 µg/ml, even more preferably between 15 µg/ml and 200 µg/ml. Preferably, said solvent is water.

Preferably, said mucoadhesive agent is glycerol, more preferably at a concentration between 0.05 % w/v and 0.2% w/v, more preferably of 0.1% w/v.

Preferably, said antioxidant is methionine, more preferably at a concentration between 0.005 mg/ml and 0.02 mg/ml, more preferably of 0.01 mg/ml.

Preferably said surfactant is Kolliphor P188, more preferably at a concentration between 0.05 % w/v and 0.2% w/v, more preferably of 0.1% w/v.

Preferably, said buffer is phosphate buffer.

Preferably, said penetration enhancer is n-Dodecyl-β-D-maltoside, more preferably at a concentration between 0.1 % w/v and 1% w/v, more preferably of 0.5% w/v.

A particularly preferred liquid intranasal composition according to the invention comprises, preferably consists of, said NGF or mutein, sodium chloride, phosphate buffer and water.

Another particularly preferred liquid intranasal composition according to the invention comprises, preferably consists of, said NGF or mutein as described above, sodium chloride, phosphate buffer, Kolliphor P188, L-Methionine, and water.

Another particularly preferred liquid intranasal composition according to the invention comprises, preferably consists of said NGF or mutein, sodium chloride, phosphate buffer, Kolliphor P188, L-Methionine, Glycerol, n-Dodecyl-β-D-maltoside and water.

Preferably, the liquid intranasal composition according to the invention comprises, preferably consists of, the following components:
- NGF or a mutein thereof, as described above, preferably at a concentration between 0.3 and 2 mg/ml, more preferably between 0.5 and 1.5 mg/ml,
- NaH2PO4 * H2O, preferably at a concentration between 5 and 8 mg/ml, more preferably of 6.9 mg/mL,
- NaCl, preferably at a concentration between 5 and 6.5 mg/ml, more preferably of 5.84 mg/mL,
- Kolliphor P188, preferably at a concentration between 0.05 % w/v and 0.2% w/v, more preferably of 0.1% w/v,
- L-Methionine, preferably at a concentration between 0.05 mg/ml and 0.2 mg/ml, more preferably of 0.1 mg/ml,
- Optionally, n-Dodecyl-β-D-maltoside, preferably at a concentration between 0.1 % w/v and 1% w/v, more preferably of 0.5% w/v, and/or glycerol, preferably at a concentration between 0.05 % w/v and 0.2% w/v, more preferably of 0.1% w/v,
- Water.

The pharmaceutical composition according to the invention may be suitably formulated using appropriate methods known in the art or by the method disclosed in Remington's Pharmaceutical Science (recent edition), Mack Publishing Company, Easton Pa. Preferably, the pharmaceutical composition of the invention is for use in the prevention or treatment of spasticity in a subject, as above described, wherein said composition is administered intranasally to the subject.

In a further aspect, the present invention relates to a method for the prevention or treatment of spasticity in a subject. Preferably, said method comprises intranasally administering NGF or a mutein thereof to the subject, as described above, in a therapeutically effective amount.

Preferably, in the method according to the invention, said NGF or mutein is administered as described above.

Preferably, said NGF or mutein used in the method of the invention is in form of a pharmaceutical composition, as above described.

The invention will be further described in the following examples, which do not limit the scope of the invention described in the claims.

### EXPERIMENTAL SECTION

### Example 1

The biodistribution of NGF in rat brain areas after single intranasal administration was evaluated.

A formulation containing 1.2 mg/mL of rhNGF was administered once intranasally to rats. Rats were sacrificed at different time-points corresponding to 2 hours, 4 hours, 8 hours and 24 hours after treatment.

Samples from parietal cortex, hypothalamus, thalamus, striatum, hippocampus, brainstem, frontal cortex and medial septum were collected for rhNGF quantitative determination by ELISA and cumulative absorption (2-24 hours) was calculated.

Before analysis, brain samples were homogenized in ice by ultraturrax and centrifuged for supernatants recovery. A commercial ELISA kit (RayBiotech, Catalogue ELH-BNGF) was used according to the instructions provided by the supplier. The calibration curve range for NGF determination was established at 20,5 - 5000 pg/mL.

As shown in Figure 1, NGF absorption was evident in all brain tissues, but the protein particularly concentrates in areas involved in motor activity, especially in hypothalamus and thalamus.

### Example 2

The role of NGF in counteracting spasticity and the associated loss of balance and motor coordination was evaluated in a rat chemogenetic model representative of neurons defects observed in HSP. Briefly, the selective blockade of cholinergic striatal neurons was achieved by stereologically injecting Cre-dependent adeno-associated virus (AAV-hSynDIO-hM4Di-mCherry) into relevant brain areas and the neuronal deactivation was achieved by clozapine-N-oxide (CNO) treatment acting on the specific promoter used to selectively modulate the functionality of cholinergic striatal neurons, as described in Aldrin-Kirk et al, Neurobiology of Disease 2018 (109), pages 148-162.

In this experiment, the selective contribution of cholinergic neurons in the development of motor dysfunctions was evaluated by rotaroad test. In addition, the functional relevance of a possible recovery in cortico-striatal connectivity promoted by intranasal administration of rhNGF (50µg/kg for 3 days) was demonstrated.

As shown in Figure 2, the blockade of cholinergic neurons (ChAT off) reduced the rotarod latency time to about 50% of that measured for the sham animals. The treatment with NGF (ChAT off + NGF) restored the motor balance to about 75% of that measured for the sham animals. No relevant mobility changes were observed in sham animals treated with NGF (Sham+ NGF). Measurements were performed 24 hours after the last treatment.

### Example 3

The effect of NGF was also evaluated in a mouse model of TBI. Animal care was carried out according to Italian (D.L. 116/92) and European Commission (O.J. of E.C. L358/1 18/12/86) regulations on the protection of laboratory animals. All efforts were made to reduce both animal numbers and suffering during the experiments.

Male C57BL/6 mice (Envigo, Italy) weighing 18-20 g were lightly anesthetized with isoflurane and placed in a prone position on a spongy support. The head was not fixed. To establish a TBI, a weight of 120 g was dropped from a height of 25 cm on mice skulls to induce a consistent injury in the brain able to establish a severe motor dysfunction. Sham mice were submitted to the same procedure as described for TBI mice, but without release of the weight.

Mice were visually observed for signs of spasticity symptoms in the limbs such as clonus (repeated muscle jerks), prolonged spasms, twitches (fast involuntary contractions) and hyperreflexia.

Treatment with NGF (50µg/kg/day for 3 days) started when spasticity symptoms were well established.

The ability of NGF to reduce spasticity and restore the muscle functionality was evaluated as improvement of motor coordination impairment by the rotarod test. The test consisted in two session of walking on a rotating cylinder, separated by a 1-hour pause. After a 30-seconds adaptation, rotation speed was gradually increased from 3 to 30 rpm, for maximum test duration of 5 minutes. Latency to fall (seconds) was recorded and final data were expressed as percentage of time latency versus sham. As reported in Figure 3, the induction of TBI (TBI) reduced the rotarod latency time to about 60% that of the sham animals. The treatment with NGF (TBI+NGF) was able to restore the motor balance to about 80% of that of the sham animals. No effect was observed of NGF per se in the treatment of sham animals (Sham+ NGF). Measurements were performed 24 hours after the last treatment.

### Example 4

Male C57BL/6 mice (Envigo, Italy) weighing 18-20 g were housed three per cage under controlled illumination (12 h light/dark cycle; light on 6:00 A.M.) and standard environmental conditions (ambient temperature 20-22°C, humidity 55-60%) for at least 1 week before to start the experiments. Mice chow and tap water were available ad libitum. Animal care was carried out according to Italian (D.L. 116/92) and European Commission (O.J. of E.C. L358/1 18/12/86) regulations on the protection of laboratory animals. All efforts were made to reduce both animal numbers and suffering during the experiments.

Severe TBI through Marmarou's weight drop model, commonly associated with spasticity symptoms, was induced according to the following protocol.

Mice were lightly anesthetized with isoflurane and placed in a prone position on a spongy support. The head was not fixed. After a midline longitudinal incision, the skull was exposed to locate the area of impact and placed under a metal tube device where the opening is positioned directly over the animal's head. The injury was induced by dropping a cylindrical metal weight (250 g), through a vertical metal guide tube from a height of 2 cm, based on Khalin and colleague's method (Khalin I et al., (2016), Neural Regen Res 11(4):630-635.). The point of impact was between the anterior coronal suture (Bregma) and posterior coronal suture (Lambda). Immediately following injury, the skin was closed with surgical wound clips and mice were placed back in their cages to allow for recovery from the anesthesia. Sham mice were submitted to the same procedure as described for TBI, but without release of the weight.

Mice were visually observed for signs of spasticity symptoms such as clonus (repeated muscle jerks), prolonged spasms, twitches (fast involuntary contractions) and hyperreflexia. Mice exhibited a spastic hypertonia starting from 24-48 hours after the injury.

Mice were treated daily with vehicle or with rhNGF, administered once daily by intranasal route starting on day 1 or day 7 until day 28 post-TBI.

In details, the animals were divided in following groups:
- Sham (n=20),
- TBI+Vehicle: treated with 20 µl of vehicle daily (TBI/vehicle; n=20),
- TBI+rhNGF: treated with rhNGF at 50 µg/Kg daily, starting at 1 day post-TBI (TBI/rhNGF d1; n=20),
- TBI+rhNGF: treated with rhNGF at 50 µg/Kg daily, starting at 7 days post-TBI (TBI/rhNGF d7; n=20),

Administration of vehicle or rhNGF was carried out as follows. Using a dominant hand, the micropipette P20 was leaded with 10 µl of compound or vehicle. The tip of the filled pipette was placed near the mouse's left nostril at 45 degree angle. The droplet was placed close enough to the mouse's nostril so that the mouse can inhale the droplet. Immediately after the mouse inhaled this small drop, the rest of the liquid in the pipette tip was ejected to form another small droplet for the mouse to inhale through the same nostril about 2-3 sec later. After administration, the mouse was held in this position for 15 sec.

Different behavioural tests, as described below, were performed in order to assess motor and locomotor impairment at different time points after TBI induction.

Data were analysed using GraphPad Prism version 8.04 (GraphPad Software). Data from behavioural experiments were expressed as mean ± SEM. Two-way ANOVA followed by Tukey's post hoc tests was used to analyse differences between groups, using treatment (drugs or Vehicle), and testing time (1, 3, 7, 14, 21 and 28 days post-TBI) as factors in the analysis.

### - Pole test

A 50 cm vertical steel pole placed in the cage and covered with adhesive tape to create a rough surface was used in the test. Animals were placed head-up on top of the pole. The time to orient downward and total time to descend were measured.

The results are shown in Figures 4a and 4b. As can be seen, TBl/vehicle mice required more time to turn and less time to descend the pole than Sham.

Intranasal rhNGF (50 ug/Kg), administered at both 1 day or 7 days post TBI significantly improved the pole test performance in a time-dependent manner, with a more pronounced effect on time to go down.

Unexpectedly, when treatment was started at day 7 it produced a greater improvement than the early treatment at day 1, completely normalizing the time to go down, which is the most significant parameter for assessing impairment of motor activity associated to spasticity. This is surprising in view of the general consensus that early treatment should be more effective in preventing secondary damages to TBI and establishment of spasticity. These data support that treatment with NGF is more effective if started once the spasticity symptoms have fully developed. This time point in the mouse corresponds to about 6-10 months after the traumatic event in case of a human subject (Dutta et al, Life Sciences 2016 (152), pages 244-248).

### - Open field test

The open field test apparatus was a large cubic box, measuring 1 m long × 1 m wide × 1 m high, wherein the top of the cube is left uncovered. An animal is placed in the middle of the bottom surface, and its movements are recorded over the course of minutes to hours as it moves around and explores its environment. After the experiment is completed, computer tracking programs analyses the movements of the animal over time. The travelling speed and the total distance travelled were determined as a measure of the animals' motor impairment.

TBI/vehicle mice showed a significant decrease in the travelled distance (1504.1 ± 451.32 cm, p<0.0001, at day 28 post-TBI) and in the travelling speed (2.5 ± 0.86 cm/sec, p<0.0001, at day 28 post-TBI) as compared to Sham mice (4995.9 ± 1066.04 cm and 7.63 ± 1.27 cm/sec) (Figs. 5A, B and C).

By contrast, TBI/rhNGF d1 (3837.75 ± 1161.4 cm, p<0.0001 for travelled distance; 4.79 ± 1.27 cm/sec, p<0.0001 for travelling speed, at day 28 post-TBI) and TBI/rhNGF d7 mice (4779.05 ± 1479.1 cm, p<0.0001 for travelled distance; 6.19 ± 0.99 cm/sec, p<0.0001 for travelling speed, at day 28 post-TBI) showed a significant improvement in the ambulatory activity as compared to TBI/vehicle mice (Figs. 5A, B and C).

Also in this test, treatment started at day 7 unexpectedly produced a greater improvement than the early treatment at day 1, restoring the tested parameters to substantially the same values observed in sham animals.

### - Von Frey

Mechanical allodynia is measured with a series of calibrated von Frey nylon filaments (Stoelting, Wood Dale, IL, USA), ranging from 0.002 to 2 g, by using a modified version of the up-down method adapted from Chaplan et al. (Chaplan et al., (1994), J. Neurosci. Methods 53, 55- 63).

The elevated mesh platform for mechanical allodynia used in the test was a large testing surface (perforated metal platform) with plexiglass boxes for animal acclimation before behavioural analysis.

The manually used von Frey filaments for mechanical allodynia were nylon monofilaments that provide an approximately logarithmic scale of actual force and a linear scale of perceived intensity. When the tip of a fiber of given length and diameter is pressed against the skin at right angles, the force of application increases as long as the researcher continues to advance the probe until the fiber bends.

The results reported in Figure 6 showed a significant decrease of paw withdrawal response (g) was observed in TBl/vehicle mice from 7 to 28 days after trauma induction (0.26 ± 0.28 g, p<0.0001, at day 28 post-TBI) compared to the sham group (1.29 ± 0.49 g); interestingly, in TBI/rhNGF d1 (0.98 ± 0.55 g, p=0.0001) and TBI/rhNGF d7 (1.35 ± 0.49 g, p<0.0001) the mechanical allodynia was significantly reduced as compared with TBl/vehicle animals.

### - Footprint test

The footprint apparatus consisted of a runway, usually 50 ~ 60 cm length, with a dark goal-box at the end. After administrating drugs, the forepaws and hind paws of the mice were painted with different colours (e.g., forepaws with red and hind paws with green). Then the mice were made to walk on the blotting paper.

TBl/vehicle mice displayed an abnormal pattern in the stride length (3.19 ± 1.15 cm, p=0.069, at day 28 post-TBI) (Figs. 7A and B), in the sway length (1.03 ± 0.51 cm, p<0.0001, at day 28 post-TBI) (Figs. 7A and C) and in the overlap (0.26 ± 0.18 cm, p=0.001, at day 28 post-TBI) (Figs. 7A and D). These step sequence irregularities were the result of spastic contractions that were interfering with a regular gait progression.

In the TBI/vehicle mice, joint movements appeared rigid with frequent trajectory changes in the swing phase, and steps were short with frequent cross-positioning of the forepaws. On the contrary, treatment of mice starting at day 7 (TBI/rhNGF d7) completely normalized the gait pattern, with an increased stride length (5.59 ± 1.58 cm, p=0.0064, at day 28 post-TBI), sway length (2.76 ± 0.69 cm, p<0.0001, at day 28 post-TBI) and improvement of overlap (1.05 ± 0.19 cm, p<0.0001, at day 28 post-TBI) (Figs. 7A-D).

Surprisingly, no significant improvements were observed in this test with early treatment with NGF starting at 1 day post TBI (TBI/rhNGF d) (Fig. 7A-D).

### Example 5

In order to assess the efficacy of intermittent intranasal administration of NGF on spasticity-associated motor impairment induced by a traumatic event and explore the effect of a longer treatment period, the same severe TBI mouse model described in Example 4 was used in further experiments.

Mice were treated for 61 days with vehicle or with rhNGF (50 ug/Kg) administered by intranasal route starting on day 7 post-TBI, for intermittent period of treatments of three consecutive days alternated to 7 days wash-out periods, until day 61 post TBI.

In details, the animals were divided in following groups:
- Sham: (Sham/Veh; n=20),
- Sham+ NGF: (50 ug/Kg) started 7 days post-TBI (Sham/rhNGF; n=20),
- TBl+Vehicle: (20 µl) (TBI/veh; n=20),
- TBI+rhNGF: (50 ug/Kg) started 7 days post-TBI (TBI/rhNGF d7; n=20).

Intranasal administration of rhNGF and vehicle was carried out as described in Example 4. The same behavioural tests as described in Example 4 were carried out. In all tests, intermittent treatment with rhNGF resulted in an improvement in the tested parameters at all time points up to about 40 days. However, after this time point, it was observed that the impairment of motor activity associated to spasticity in this animal model spontaneously reverted versus the basal level and therefore results lost significance. The results obtained demonstrate that prolonged administration of rhNGF by intermittent administration is effective in treating spasticity.

Figures 8 shows representative results obtained in the Pole test.

## Claims

1. Nerve growth factor (NGF) or a mutein thereof, said mutein having the amino acid sequence of SEQ ID NO:3, SEQ ID NO:4, SEQ ID NO:5 or SEQ ID NO:6, for use in the prevention or treatment of spasticity in a subject, wherein said NGF or mutein is administered intranasally to said subject.

2. NGF or mutein for use as claimed in claim 1, wherein said spasticity is caused by a condition selected from cerebral palsy, stroke, traumatic brain injury, amyotrophic lateral sclerosis, primary lateral sclerosis, multiple sclerosis (MS) and hereditary spastic paraplegia (HSP).

3. NGF or mutein for use as claimed in claims 1 or 2, wherein said NGF is human NGF, more preferably it is recombinant human NGF.

4. NGF or mutein for use as claimed in claim 3, wherein said human NGF has the amino acid sequence of SEQ ID NO:1.

5. NGF or mutein for use as claimed in claims 1 to 4, wherein said NGF or mutein is administered from one to three times a day for a period of treatment of between 7 and 300 days, preferably between 60 and 240 days, more preferably between 100 and 200 days.

6. NGF or mutein for use as claimed in claims 1 to 5, wherein the amount of NGF or mutein per each administration is between 5 µg and 1 mg, more preferably between 10 µg and 400 µg, even more preferably between 15 µg and 200 µg.

7. A pharmaceutical composition for intranasal administration comprising NGF or a mutein thereof, said mutein having the amino acid sequence of SEQ ID NO:3, SEQ ID NO:4, SEQ ID NO:5 or SEQ ID NO:6, and at least one pharmaceutically acceptable excipient, for use in the prevention or treatment of spasticity in a subject, wherein said NGF or mutein is administered intranasally to said subject.

8. A pharmaceutical composition for use as claimed in claim 7, wherein said spasticity is caused by a condition selected from cerebral palsy, stroke, traumatic brain injury, amyotrophic lateral sclerosis, primary lateral sclerosis, multiple sclerosis (MS) and hereditary spastic paraplegia (HSP).

9. A pharmaceutical composition for use as claimed in claim 7 or 8, wherein said NGF or mutein thereof is present in the composition at a concentration between 5 µg/ml and 1 mg/ml, more preferably between 10 µg/ml and 400 µg/ml, even more preferably between 15 µg/ml and 200 µg/ml.

10. A pharmaceutical composition for use as claimed in claims 7 to 9, comprising, preferably consisting of NGF or mutein thereof, sodium chloride, phosphate buffer and water.

11. A pharmaceutical composition for use as claimed in claims 7 to 10, wherein said NGF is human NGF, more preferably it is recombinant human NGF.

12. A pharmaceutical composition for use as claimed in claim 7, wherein said human NGF has the amino acid sequence of SEQ ID NO:1 or SEQ ID NO:2.

13. NGF or mutein for use as claimed in claims 1 to 6, wherein said spasticity in a subject is caused by stroke or traumatic brain injury, and said NGF or mutein is administered to said subject starting between 6 and 10 months, more preferably between 8 and 10 months, more preferably between 9 and 10 months, even more preferably at 9 months after from the occurrence of said stroke or traumatic brain injury.
